# EUROPEAN PATENT SPECIFICATION

(11) **EP 0 856 773 B1**
(45) Date of publication and mention of the grant of the patent: **13.06.2001**
(21) Application number: 98101371.7
(22) Date of filing: 27.01.1998
(51) Int. Cl.: G03F 7/039, G03F 7/004

(54) **Chemical amplification type positive resist composition**
Chemisch verstärkte Positiv-Resistzusammensetzung
Compostion formant réserve postive avec amplification chimique

(30) Priority: 29.01.1997 JP 1535397
(43) Date of publication of application: 05.08.1998
(73) Proprietor: Sumitomo Chemical Company, Limited, Chuo-ku Osaka 541-8550 (JP)
(72) Inventor: Uetani, Yasunori, Toyonaka-shi, Osaka (JP); Fujishima, Hiroaki, Toyonaka-shi, Osaka (JP); Miya, Yoshiko, Muko-shi, Kyoto (JP)
(74) Representative: VOSSIUS & PARTNER

(56) References cited:
- WO-A-95/03310
- US-A- 5 674 662
- DATABASE WPI Section Ch, Week 9326 Derwent Publications Ltd., London, GB; Class A89, AN 93-208243 XP002064083 & JP 04 251 849 A (FUJI PHOTO FILM CO., LTD.) , 8 September 1992
- PATENT ABSTRACTS OF JAPAN vol. 14, no. 398 (P-1097), 28 August 1990 & JP 02 149848 A (OKI ELECTRIC IND. CO., LTD.), 8 June 1990,

## Description

The present invention relates to a chemical amplification type positive resist composition used for a fine processing in the production of semiconductors.

A lithography process using a resist composition is normally employed for a fine processing in the production of semiconductors. In the lithography process, as shown by Rayleigh's formula of diffraction limit, the shorter the exposure wavelength, the more the resolution can be increased in principle. Regarding the exposure light source for lithography used for producing semiconductors, its wavelength is decreasing every year. That is, g-ray with a wavelength of 436 nm, which had been widely used, was replaced by i-ray with a wavelength of 365 nm and recently, i-ray is being replaced by KrF excimer laser with a wavelength of 248 nm. As the exposure light source for the next generation, ArF excimer laser with a wavelength of 193 nm is expected.

Since a lens used in an ArF excimer laser aligner has a life time shorter than that of an aligner using a conventional exposure light source, shorter time for exposure of ArF excimer laser is desired. It is necessary to enhance the sensitivity of a resist to decrease the exposure time. Therefore, a so-called chemical amplification type resist, which utilizes a catalytic action of the acid generated by the exposure and contains a resin having a group cleavable by an action of the acid, is preferably used.

It is known that a resin having no aromatic ring but having an alicyclic ring in place of the aromatic ring is preferred for using in the resist for ArF excimer laser exposure in order to secure light transmission of the resist and to impart dry etching resistance. As such a resin, various resins, such as those described in D.C. Hofer, Journal of Photopolymer Scince and Technology, Vol. 9, No. 3 (1996) 387-398, have hitherto been known. However, the known resins have a problem that peeling at the time of developing is liable to be caused due to lack of adhesion at developing, particularly when the resin lacks polarity.

An object of the present invention is to provide a chemical amplification type positive resist composition comprising a resin component and an acid generator, which is suitable for excimer laser (e.g. ArF laser, KrF laser, etc.) lithography, and which is good in various resist performances such as sensitivity and resolution and is particularly good in adhesion to a substrate.

This object could be achieved on the basis of the finding that the adhesion of a chemical amplification type positive resist composition to a substrate is improved by introducing a certain group into a resin comprised in the resist composition.

The present invention provides a chemical amplification type positive resist composition comprising a resin having a butyrolactone residue which may be optionally substituted with an alkyl group as well as a group capable of cleaving by action of an acid, and an acid generator.

The resin comprised in the resist composition of the present invention is normally produced by polymerizing or copolymerizing a compound having an ethylenically unsaturated bond. It is preferred that the resin has no aromatic ring but has an alicyclic ring. The alicyclic ring is preferably an alicyclic hydrocarbon residue, particularly an alicyclic hydrocarbon residue having a crosslinked hydrocarbon ring. Examples of the alicyclic ring include a bornane ring, norbornane ring, tricyclodecane ring, tetracyclododecane ring and adamantane ring. Examples of the compound having an ethylenically unsaturated bond, i.e. the monomer unit from which the resin is produced, include a compound derived from an alicyclic ester of (meth)acrylic acid and a compound derived from a vinyl or isopropenyl ester of an alicyclic carboxylic acid.

The resin comprised in the resist composition of the present invention has a butyrolactone residue in it. The butyrolactone residue includes not only an unsubstituted butyrolactone residue but also a butyrolactone residue substituted with an alkyl group. Example of the alkyl group include those having about 1 to 4 carbon atoms, i.e. methyl, ethyl, propyl, and butyl groups. The butyrolactone residue may be linked to the resin base via an ester or ether linkage. The position in the butyrolactone residue which links to the resin base is not specifically limited. For example, it may be the a-position (i.e. 2-position) of butyrolactone.

The resin comprised in the resist composition of the present invention may comprise both a polymerized unit which has an alicyclic ring and a polymerized unit which has a butyrolactone residue. The resin may also comprise a polymerized unit which has both an alicyclic ring and a butyrolactone residue. For example, the polymerized unit may be derived from the alicyclic ester of (meth)acrylic acid, wherein the butyrolactone residue is linked to the alicyclic ring via an ester or ether linkage.

The butyrolactone residue may be introduced into the resin base after the resin base was produced, or may be introduced into the monomer unit which is, then, polymerized to produce the resin.

The butyrolactone residue may be introduced into the resin or the monomer unit, for example, by converting a carboxylic group present in the resin into a butyrolactone ester or converting an alcoholic hydroxyl group present in the resin into a butyrolactone ether. In the esterification or etherification, for example, there can be used a halogen-substituted substance of butyrolactone which may be optionally substituted with an alkyl group. Examples of the monomer unit into which a butyrolactone residue is introduced include α-acryloyloxy-γ-butyrolactone, α-methacryloyloxy-γ-butyrolactone, α-acryloyloxy-β and β-dimethyl-γ-butyrolactone.

The resin for chemical amplification type resist is insoluble or slightly soluble in an alkaline environment as it is, but a part of groups is cleaved by an action of an acid and the resin becomes alkaline-soluble after cleaving. Therefore, the resin used in the present invention has a group capable of cleaving by an action of an acid, in addition to the butyrolactone residue. The group capable of cleaving by action of an acid may contain an alicyclic ring mentioned above.

The group capable of cleaving by action of an acid is known in the field of resists. Examples thereof include alkyl groups branched at the 1-position, such as tert-butyl; optionally substituted 1-alkoxyalkyl groups such as 1-ethoxyethyl, 1-(2-methoxyethoxy)ethyl, 1-(2-acetoxyethoxy)ethyl, 1-[2-(1-adamantyloxy)ethoxy]ethyl and 1-[2-(1-adamantanecarbonyloxy)ethoxy]ethyl; and residues of non-aromatic cyclic compounds, such as tetrahydro-2-furanyl, tetrahydro-2-pyranyl, 3-oxocyclohexyl, 4-methyltetrahydro-2-pyron-4-yl (derived from mevalonic lactone) and 2-methyl-2-adamantyl.

The group capable of cleaving by action of an acid, such as those mentioned above, are introduced into the resin or a monomer unit from which the resin is produced by substituting hydrogen in the carboxylic group or alcoholic hydroxyl group of the resin or monomer unit with these groups. The monomer unit into which such a group was introduced may be copolymerized with the other monomer to produce the resin used in the present invention.

In general, the resin used in the present invention preferably contains a polymerized unit having a butyrolactone residue in an amount within the range from 10 to 60% by mol of the total of polymerized units, although the range varies depending on the kind of radiation for patterning exposure and the kind of the group capable of cleaving by action of an acid. The amount of the group capable of cleaving by action of an acid is preferably within the range from 10 to 90% by mol of the total of polymerized units. The polymerized units having an alicyclic ring, which may or may not have the groups capable of cleaving by action of an acid simultaneously, is preferably present in an amount of not less than 20% by mol of the total of polymerized units. This resin may also contains the other polymerized unit such as free carboxylic group as far as the effect of the present invention is not adversely affected.

The acid generator, another component of the resist composition of the present invention, is a compound which is decomposed to generate an acid by exposing the compound itself or a resist composition containing the compound to radiation such as light and electron beam. The acid generated from the acid generator reacts with the resin, thereby to cleave the group capable of cleaving by action of an acid. Examples of the acid generator include onium salt compounds, organohalogen compounds, sulfone compounds and sulfonate compounds. Specific examples thereof include the following compounds: diphenyliodonium trifluoromethanesulfonate, 4-methoxyphenylphenyliodonium hexafluoroantimonate, 4-meythoxyphenylphenyliodonium trifluoromethanesulfonate, bis(4-tert-butylphenyl)iodonium tetrafluoroborate, bis(4-tert-butylphenyl)iodonium hexafluorophosphate, bis(4-tert-butylphenyl)iodonium hexafluoroantimonate, bis(4-tert-butylphenyl)iodonium trifluoromethanesulfonate, triphenylsulfonium hexafluorophosphate, triphenylsulfonium hexafluoroantimonate, triphenylsulfonium trifluoromethanesulfonate, 4-methoxyphenyldiphenylsulfonium hexafluoroantimonate, 4-methoxyphenyldiphenylsulfonium trifluoromethanesulfonate, p-tolyldiphenylsulfonium trifluoromethanesulfonate, 2,4,6-trimethylphenyldiphenylsulfonium trifluoromethanesulfonate, 4-tert-butylphenyldiphenylsulfonium trifluoromethanesulfonate, 4-phenylthiophenyldiphenylsulfonium hexafluorophosphate, 4-phenylthiophenyldiphenylsulfonium hexafluoroantimonate, 1-(2-naphthoylmethyl)thiolanium hexafluoroantimonate, 1-(2-naphthoylmethyl)thiolanium trifluoromethanesulfonate, 4-hydroxy-1-naphthyldimethylsulfonium hexafluoroantimonate, 4-hydroxy-1-naphthyldimethylsulfonium trifluoromethanesulfonate, 2-methyl-4,6-bis(trichloromethyl)-1,3,5-triazine, 2,4,6-tris(trichloromethyl)-1,3,5-triazine, 2-phenyl-4,6-bis(trichloromethyl)-1,3,5-triazine, 2-(4-chlorophenyl)-4,6-bis(trichloromethyl)-1,3,5-triazine, 2-(4-methoxyphenyl)-4,6-bis(trichloromethyl)-1,3,5-triazine, 2-(4-methoxy-1naphthyl)-4,6-bis(trichloromethyl)-1,3,5-triazine, 2-(benzo[d][1,3]dioxolan-5-yl)-4,6-bis(trichloromethyl)-1,3,5-triazine, 2-(4-methoxystyryl)-4,6-bis(trichloromethyl)-1,3,5-triazine, 2-(3,4,5-trimethoxystyryl)-4,6-bis(trichloromethyl)-1,3,5-triazine, 2-(3,4-dimethoxystyryl)-4,6-bis(trichloromethyl)-1,3,5-triazine, 2-(2,4-dimethoxystyryl)-4,6-bis(trichloromethyl)-1,3,5-triazine, 2-(2-methoxystyryl)-4,6-bis(trichloromethyl)-1,3,5-triazine, 2-(4-butoxystyryl)-4,6-bis(trichloromethyl)-1,3,5-triazine, 2-(4-pentyloxystyryl)-4,6-bis(trichloromethyl)-1,3,5-triazine, 1-benzoyl-1-phenylmethyl p-toluenesulfonate, 2-benzoyl-2-hydroxy-2-phenylethyl p-toluenesulfonate, 1,2,3-benzenetoluyl trimethanesulfonate, 2,6-dinitrobenzyl p-toluenesulfonate, 2-nitrobenzyl p-toluenesulfonate, 4-nitrobenzyl p-toluenesulfonate, diphenyl disulfone, di-p-tolyl disulfone, bis (phenylsulfonyl)diazomethane, bis(4-chlorophenylsulfonyl)diazomethane, bis (p-tolylsulfonyl)diazomethane, bis(4-tert-butylphenylsulfonyl) diazomethane, bis(2,4-xylylsulfonyl)diazomethane, bis(cyclohexylsulfonyl)diazomethane, (benzoyl) (phenylsulfonyl)diazomethane, N-(phenylsulfonyloxy)succinimide, N-(trifluoromethylsulfonyloxy)succinimide, N-(trifluoromethylsulfonyloxy)phthalimide, N-(trifluoromethylsulfonyloxy)-5-norbornene-2,3-dicarboxyimide, N-(trifluoromethylsulfonyloxy)naphthalimide and N-(10-camphorsulfonyloxy)naphthalimide.

It is known that, in the chemical amplification type positive resist composition, the performance deterioration due to deactivation of the acid caused during standing after exposure can be generally reduced by adding a basic compound, particularly basic nitrogen-containing organic compound such as amines as a quencher. In the present invention, it is also preferred that such a basic compound is blended in the resist composition. Specific examples of the basic compound used as the quencher include those represented by the following respective formulas: 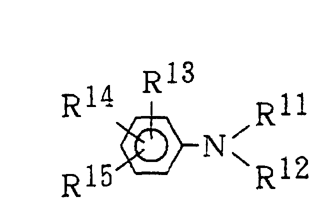 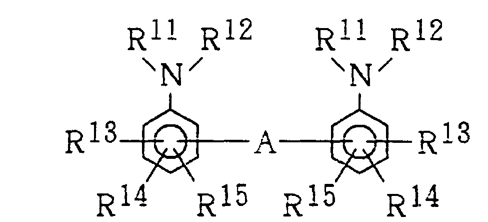 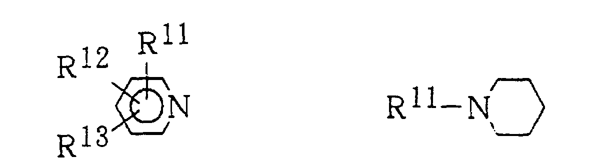 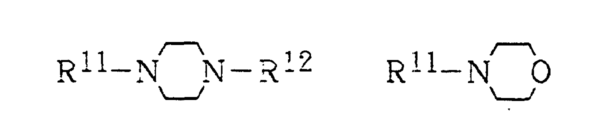 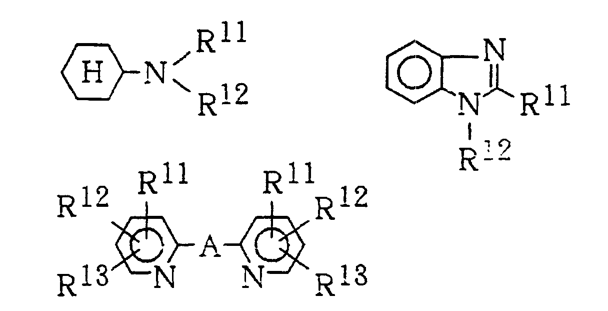 wherein R¹¹, R¹², R¹³, R¹⁴ and R¹⁵ independently represent hydrogen, or an alkyl, cycloalkyl, aryl or alkoxy group which may be substituted with a hydroxyl group; and A represents alkylene, carbonyl or imino. The alkyl or alkoxy represented by R¹¹ to R¹⁵ may have about 1 to 6 carbon atoms. The cycloalkyl represented by R¹¹ to R¹⁵ may have about 5 to 10 carbon atoms, and the aryl represented by R¹¹ to R¹⁵ may have about 6 to 10 carbon atoms. The alkylene represented by A may have about 1 to 6 carbon atoms, and may be straight-chain or branched.

The resist composition of the present invention preferably comprises
the resin having a butyrolactone residue which may be optionally substituted with an alkyl as well as a group capable of cleaving by action of an acid in an amount within the range from 80 to 99.9 and
an acid generator in an amount within the range from 0.1 to 20% by weight,
based on the weight of the whole solid content. When using a basic compound as the quencher, the resin composition preferably contains the basic compound in an amount within the range from 0.0001 to 0.1% by weight based on the weight of the whole solid content of the resist composition. This composition may also contain a small amount of various additives such as sensitizers, solubility inhibitors, other resins, surfactants, stabilizers, or dyes, if necessary.

The above respective components in the resist composition of the present invention are normally dissolved in a solvent to produce a resist solution, which is applied on a substrate such as a silicon wafer. The solvent used herein may be any one which dissolves the respective components and has a suitable evaporation rate, and also imparts an uniform smooth film after evaporation of the solvent. This solvent may be a solvent which is normally used in this field. Examples thereof include glycol ether esters such as ethylcellosolve acetate, methylcellosolve acetate and propylene glycol monomethyl ether acetate; esters such as ethyl lactate, butyl acetate, amyl acetate and ethyl pyruvate; ketones such as acetone, methyl isobutyl ketone, 2-heptanone and cyclohexanone; and cyclic esters such as γ-butyrolactone. These solvents may be use singly or in combination thereof.

A resist film obtained by applying a resist solution containing a resist composition of the present invention on a substrate and drying the resist solution is subjected to an exposure treatment for patterning, subjected to a heat treatment for accelerating a deblocking group reaction and then developed with an alkaline developer. The alkaline developer used herein may be various aqueous alkaline solutions used in this field, although an aqueous solution of tetramethylammonium hydroxide is widely used.

### EXAMPLES

The present invention will now be illustrated by means of the following Examples, which should not be construed to limit the scope of the invention. In the following Examples, "parts" are by weight unless otherwise stated.

### Synthesis Example 1 (synthesis of a monomer)

α-bromo-γ-butyrolactone (100 g) and methacrylic acid (104.4 g; i.e. 2.0 mol per mol of α-bromo-γ-butyrolactone) were charged, and, thereto, methyl isobutyl ketone was added in an amount which is three times by weight as that of α-bromo-γ -butyrolactone to prepare a solution. To the solution, triethylamine (183.6 g, i.e. 3.0 mol per mol of α-bromo-γ-butyrolactone) was added dropwise, followed by stirring at room temperature for about 10 hours. After the resulting reaction mixture was filtered, the organic layer was washed with an aqueous 5 % by weight sodium hydrogencarbonate solution and then washed twice with water. Thereafter, the organic layer was concentrated to obtain α-methacryloxy-γ-butyrolactone represented by the following formula in yield of 85%.

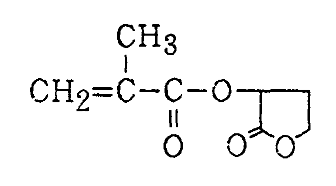

### Synthesis Example 2 (synthesis of another monomer)

1-Adamantanecarbonyl chloride (55.1 g) and pyridine (44 g) were charged, and, thereto, methyl isobutyl ketone was added in an amount which is two times by weight as that of 1-adamantanecarbonyl chloride to prepare a solution. To the solution, ethylene glycol monovinyl ether (37.2 g, i.e. 1.5 mol per mol of 1-adamantanecarbonyl chloride) was added dropwise, followed by stirring at room temperature for about 10 hours. After the resulting reaction mixture was filtered, the organic layer was washed with an aqueous 5 % by weight sodium hydrogencarbonate solution and then washed twice with water. After the organic layer was concentrated, a three-fold amount (by weight) 1,4-dioxane was added to prepare a solution. In the solution, 0.8 g of p-toluenesulfonic acid monohydrate and methacrylic acid (36 g) were charged, followed by stirring at room temperature for about 10 hours. Thereafter, the organic layer was washed with an aqueous 5 weight % sodium hydrogencarbonate solution and then washed twice with water. The organic layer was concentrated to obtain 1-adamantanecarboxylic acid 2-(1-methacryloyloxyethoxy)ethyl represented by the following formula in yield of 75%.

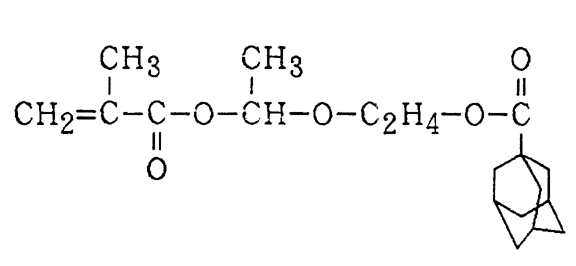

### Synthesis Example 3 (synthesis of another monomer)

2-Methyl-2-adamantanol (83.1 g) and triethylamine (101 g) were charged, and, thereto, methyl isobutyl ketone (200 g) was added to prepare a solution. To the solution, methacrylic acid chloride (78.4 g, i.e. 1.5 mol per mol of 2-methyl-2-adamanthanol) was added dropwise, followed by stirring at room temperature for about 10 hours. After the resulting reaction mixture was filtered, the organic layer was washed with an aqueous 5 weight % sodium hydrogencarbonate solution and then washed twice with water. The organic layer was concentrated and then distilled under reduced pressure to obtain 2-methyl-adamantyl methacrylate represented by the following formula in yield of 75%.

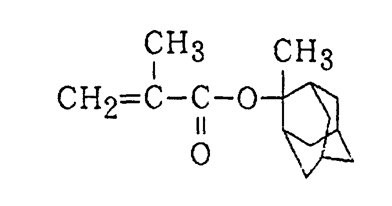

### Synthesis Example 4 4 (synthesis of resin A1)

1-ethoxyethyl methacrylate, isobornyl methacrylate and α-methacryloxy-γ-butyrolactone obtained in Synthesis Example 1 were charged in a molar ratio of 5:3:2 (31.6 g : 26.7 g : 13.6 g), respectively, and methyl isobutyl ketone was added in an amount which is two times by weight as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 50:30:20 and a weight-average molecular weight measured by GPC with 230 nm UV detector calculated as polystyrene is about 10,000, was obtained.

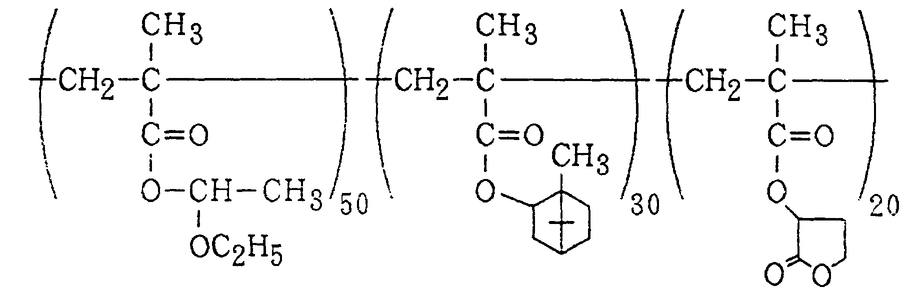

### Synthesis Example 5 (synthesis of resin AX: for comparison)

According to the same manner as that described in Synthesis Example 4 except for 13.6 g of α-methacryloxy-γ-butyrolactone was replaced with 6.9 g of methacrylic acid (the molar ratio of 1-ethoxyethyl methacrylate : isobornyl methacrylate : methacrylic acid is 5:3:2), the operation was performed. As a result, a copolymer represented by the following formula, wherein a composition molar ratio of each unit is 50:30:20 and a weight-average molecular weight measured by GPC with 230 nm UV detector calculated as polystyrene is about 10,000, was obtained.

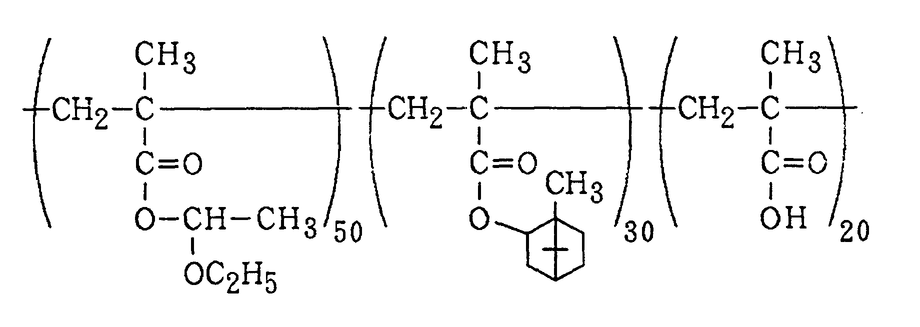

### Synthesis Example 6 (synthesis of resin A2)

1-adamantanecarboxylic acid 2-(1-methacryloyloxyethoxy)ethyl obtained in Synthesis Example 2 and α-methacryloxy-γ-butyrolactone obtained in Synthesis Example 1 were charged in a molar ratio of 8:2 (33.7 g : 4.3 g). Thereto, methyl isobutyl ketone was added in an amount which is two times by weight as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 80:20 and a weight-average molecular weight measured by GPC with 230 nmUV detector calculated as polystyrene is about 10,000, was obtained.

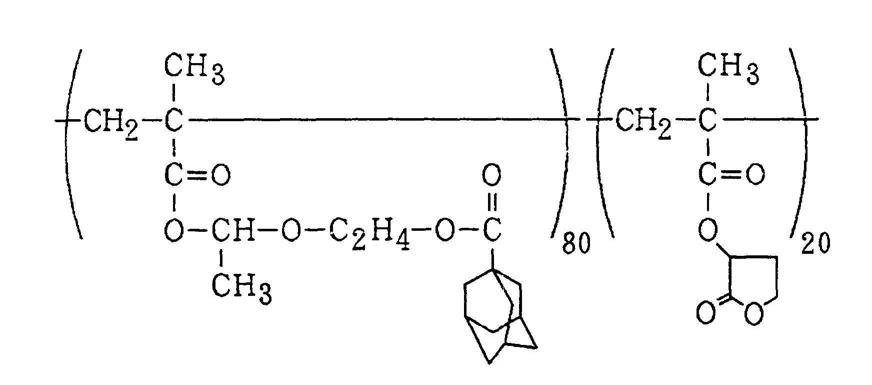

### Synthesis Example 7 (synthesis of resin AY: for comparison)

According to the same manner as that described in Synthesis Example 6 except for 4.3 g of α-methacryloxy-γ-butyrolactone was replaced with 2.2 g of methacrylic acid (the molar ratio of 1-adamantanecarboxylic acid 2-(1-methacryloyloxyethoxy)ethyl to methacrylic acid is 8:2), the operation was performed. As a result, a copolymer represented by the following formula, wherein a composition molar ratio of each unit is 80:20 and a weight-average molecular weight measured by GPC with 230 nm UV detector calculated as polystyrene is about 10,000, was obtained.

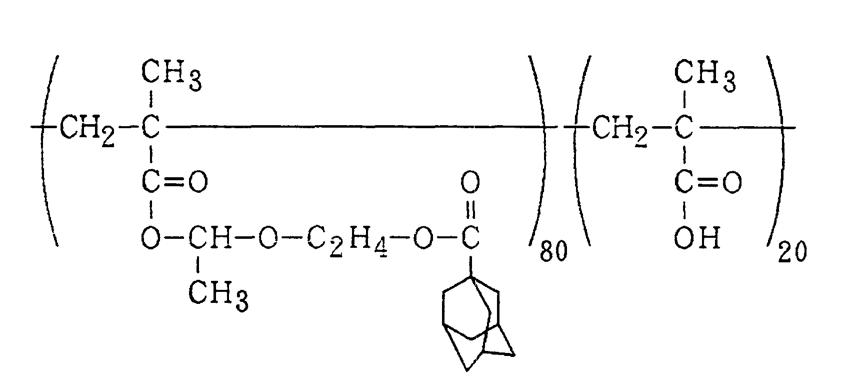

### Synthesis Example 8 (synthesis of resin A3)

2-methyl-2-adamantyl methacrylate obtained in Synthesis Example 3, 1-adamantanecarboxylic acid 2-(1-methacryloyloxyethoxy)ethyl obtained in Synthesis Example 2 and α-methacryloxy-γ-butyrolactone obtained in Synthesis Example 1 were charged in a molar ratio of 3:5:2 (23.4 g : 56.2 g : 11.3 g). Thereto, methyl isobutyl ketone was added in an amount which is two times as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 30:50:20 and a weight-average molecular weight measured by GPC with 230 nm UV detector calculated as polystyrene is about 10,000, was obtained.

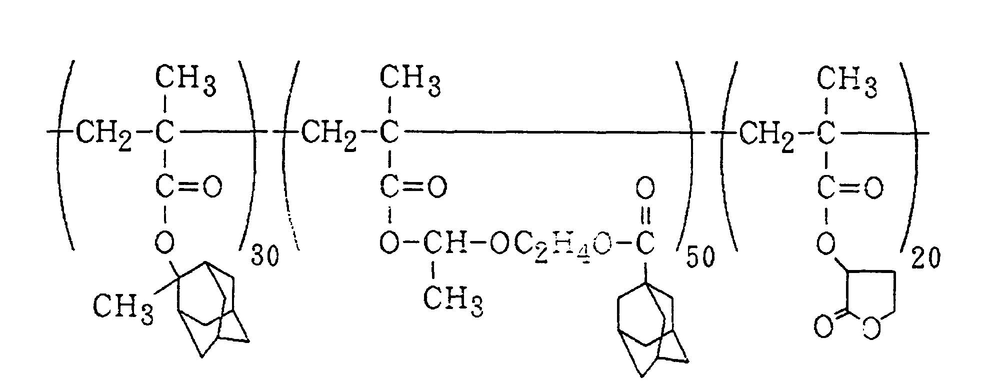

### Synthesis Example 9 (synthesis of resin AZ: for comparison)

According to the same manner as that described in Synthesis Example 8 except for 11.3 g of α-methacryloxy-γ-butyrolactone was replaced with 5.7 g of methacrylic acid (the molar ratio of 2-methyl-2-adamantyl methacrylate : 1-adamantanecarboxylic acid 2-(1-methacryloyloxyethoxy)ethyl : methacrylic acid is 3:5:2), the operation was performed. As a result, a copolymer represented by the following formula, wherein a composition molar ratio of each unit is 30:50:20 and a weight-average molecular weight measured by GPC with 230 nm UV detector calculated as polystyrene is about 10,000, was obtained.

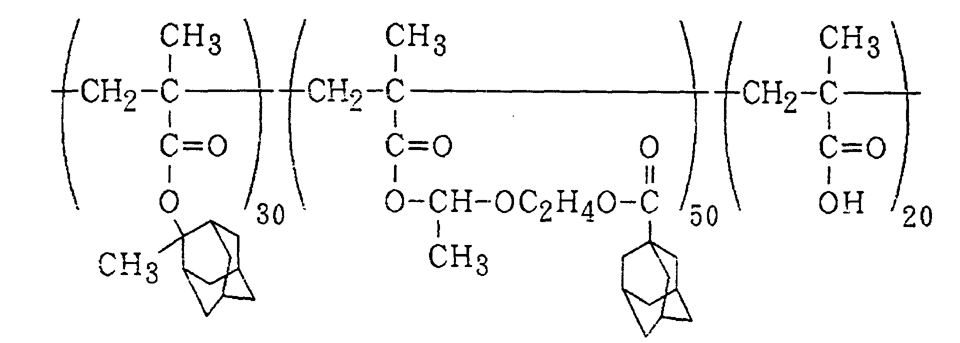

### Examples 1 to 6 and Comparative Examples 1 to 6

In each of the Examples 1 to 6 and Comparative Examples 1 to 6, 15 parts of a resin shown in Table 1, 0.15 parts of diphenyldisulfone (manufactured by Midori Kagaku Co., Ltd.) as the acid generator and 0.01 parts of a quencher shown in Table 1 were dissolved in 60 parts of propylene glycol monomethyl ether acetate, and the resulting solution was filtered through a fluororesin filter having a pore diameter of 0.2 *µ* m to prepare a resist solution. This solution was coated, on a silicon wafer (contact angle of water: 60°) treated with hexamethyldisilazane (HMDS) or on a silicon wafer on which an organic anti-reflection coating was formed, so that a film thickness after drying became 0.72 *µ* m. The organic anti-reflection coating was formed by applying "XHRi-11" manufactured by Brewer Co. and baking under the conditions of 175°C for 60 seconds so that the thickness became 1700 Å. Prebaking after coating the resist solution was performed on a direct hot plate under the conditions of 90°C for 60 seconds.

Using a KrF excimer stepper ["NSR 1755 EX8A", NA=0.45, manufactured by Nikon Co., Ltd.], the wafer on which a resist film was formed was exposed **to form a line-and-space pattern.** A post exposure baking (PEB) was performed on a hot plate under the conditions shown in Table 1 and, furthermore, dip development was performed for 60 seconds by using a developer of an aqueous 2.38 weight % tetramethylammonium hydroxide solution or a developer prepared by diluting the solution with pure water as shown in Table 1. The pattern after development was observed by using a scanning electron microscope. The sensitivity and resolution were examined according to the following method, about the pattern obtained from the resist film formed on the organic anti-reflection coating substrate. The results are shown in Table 1.

Sensitivity: It was represented by an exposure dose at which a line-and-space pattern of 0.5 *µ* m becomes 1:1 (effective sensitivity)

Resolution: It was represented by a maximum dimension of a line-and-space pattern which is separated at an exposure dose of an effective sensitivity.

Using the pattern on the substrate on which organic anti-reflection coating was not formed, evaluation of the adhesion was performed at an exposure dose at which a line-and-space pattern of 0.5 *µ* m becomes 1:1. The results are shown in Table 1, wherein those keeping good adhesion are marked "O" and those causing peeling are marked "×".

**Table 1**

| Example No. | Resin | Quencher | PEB condition | Developer | Effective sensitivity | Resolution | Adhsesion |
|---|---|---|---|---|---|---|---|
| Example 1 | A1 | C1 | 100°C x 60 sec. | 1/20 | 45 mJ/cm² | 0.35 µm | ○ |
| Example 2 | A1 | C1 | 100°C x 60 sec. | 1/5 | 40 mJ/cm² | 0.32 µm | ○ |
| Example 3 | A2 | C2 | 60°C x 60 sec. | 1/5 | 45 mJ/cm² | 0.35 µm | ○ |
| Example 4 | A2 | C2 | 60°C x 60 sec. | Not diluted | 40 muj/cm² | 0.32 µm | ○ |
| Example 5 | A3 | C2 | 70°C x 60 sec. | 1/5 | 65 mJ/cm² | 0.35 µm | ○ |
| Example 6 | A3 | C2 | 70°C x 60 sec. | Not diluted | 50 mJ/cm² | 0.31 µm | ○ |
| Comparative example 1 | AX | C1 | 100°C x 60 sec. | 1/20 | 30 mJ/cm² | 0.40 µm | ○ |
| Comparative example 2 | AX | C1 | 100°C x 60 sec. | 1/5 | 25 mJ/cm² | 0.35 µm | × |
| Comparative example 3 | AY | C2 | 60°C x 60 sec. | 1/5 | 30 mJ/cm² | 0.40 µm | ○ |
| Comparative example 4 | AY | C2 | 60°C x 60 sec. | Not diluted | 25 mJ/cm² | 0.35 µm | × |
| Comparative example 5 | AZ | C2 | 70°C x 60 sec. | 1/5 | 40 mJ/cm² | 0.40 µm | ○ |
| Comparative example 6 | AZ | C2 | 70°C x 60 sec. | Not diluted | 30 muj/cm² | 0.35 µm | × |
| (Footnote of Table 1) Quencher C1: N-phenyldiethanolamine C2: 2,6-diisopropylaniline | | | | | | | |

Developer : dilution ratio (= 2.38 weight % tetramethylammonium hydroxide solution / pure water, by weight) are shown

### Synthesis Example 10 (synthesis of resin A4)

2-methyl-2-adamantyl methacrylate obtained in Synthesis Example 3 and α-methacryloxy-γ-butyrolactone obtained in Synthesis Example 1 were charged in a molar ratio of 5:5 (40.0 g : 29.0 g). Thereto, methyl isobutyl ketone was added in an amount which is two times as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 50:50 and a weight-average molecular weight measured by GPC with 230 nm UV detector calculated as polystyrene is about 8,000, was obtained.

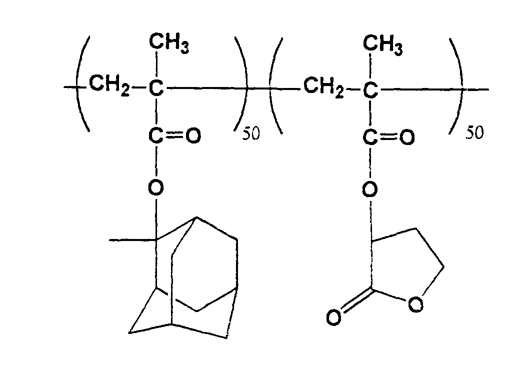

### Synthesis Example 11 (synthesis of resin A5)

2-methyl-2-adamantyl methacrylate obtained in Synthesis Example 3, α-methacryloxy-γ-butyrolactone obtained in Synthesis Example 1 and 1-ethoxyethylmethacrylate were charged in a molar ratio of 5:4:1 (40.0 g : 23.2 g : 5.4 g). Thereto, methyl isobutyl ketone was added in an amount which is two times as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 50:40:10 and a measured by GPC with 230 nm UV detector calculated as polystyrene is about 8,000, was obtained.

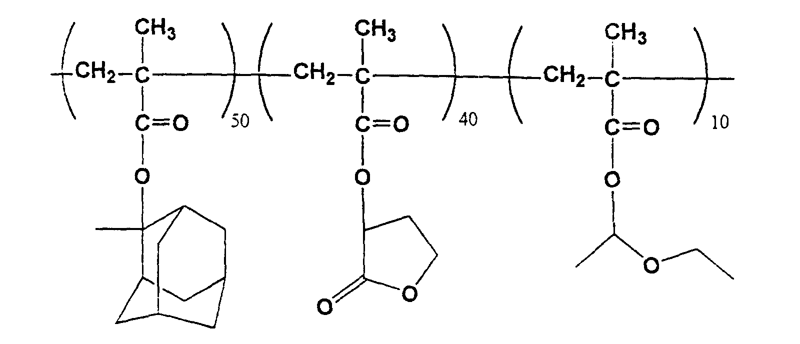

### Synthesis Example 12 (synthesis of resin A6)

2-methyl-2-adamantyl methacrylate obtained in Synthesis Example 3, α-methacryloxy-γ-butyrolactone obtained in Synthesis Example 1 and 1-isobutoxyethylmethacrylate were charged in a molar ratio of 5:4:1 (40.0 g : 23.2 g : 6.4 g). Thereto, methyl isobutyl ketone was added in an amount which is two times as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 50:40:10 and a weight-average molecular weight measured by GPC with 230 nm UV detector calculated as polystyrene is about 8,000, was obtained.

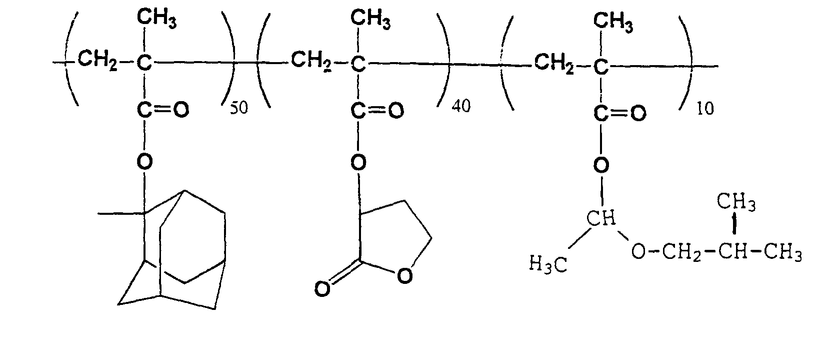

### Synthesis Example 13 (synthesis of resin A7)

2-methyl-2-adamantyl methacrylate obtained in Synthesis Example 3, α-methacryloxy-γ-butyrolactone obtained in Synthesis Example 1 and t-butylmethacrylate were charged in a molar ratio of 5:4:1 (40.0 g : 23.2 g : 4.8 g). Thereto, methyl isobutyl ketone was added in an amount which is two times as that of the whole monomer to prepare a solution. To the solution, azobisisobutyronitrile as an initiator was added in an amount of 2% by mol based on the whole monomer amount, followed by heating at 80 °C for about 8 hours. Thereafter, an operation of pouring the reaction solution into a large amount of heptane to form a precipitate was performed twice, followed by purification. As a result, a copolymer represented by the following formula, wherein a composition molar ratio of each unit is 50:40:10 and a measured by GPC with 230 nm UV detector calculated as polystyrene is about 8,000, was obtained.

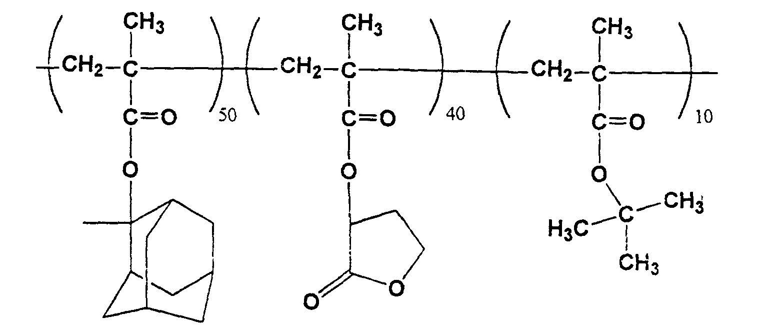

### Examples 7 to 10

In each of the Examples 7 to 10, 15 parts of a resin shown in Table 1, 0.30 parts of 4-methylphenyldiphenylsulfonium trifluoromethanesulfonate (manufactured by Midori Kagaku Co., Ltd.) as the acid generator and 0.02 parts of a quencher shown in Table 1 were dissolved in 70 parts of propylene glycol monomethyl ether acetate, and the resulting solution was filtered through a fluororesin filter having a pore diameter of 0.2 *µ* m to prepare a resist solution. This solution was coated, on a silicon wafer (contact angle of water: 60° ) treated with hexamethyldisilazane (HMDS) or on a silicon wafer on which an organic anti-reflection coating was formed, so that a film thickness after drying became 0.455 *µ* m. The organic anti-reflection coating was formed by applying "DUV-18L" manufactured by Brewer Co. and baking under the conditions of 215°C for 60 seconds so that the thickness became 570 Å. Prebaking after coating the resist solution was performed on a direct hot plate under the conditions of 130°C for 60 seconds.

Using the resist film thus obtained, according to conditions shown in Table 2 and according to same manner as in Example 1-6, the sensitivity and resolution were examined and evaluation of the adhesion was performed. The results are shown in Table 2.

**Table 2**

| Example No. | Resin | Quencher | PEB condition | Developer | Effective sensitivity | Resolution | Adhesion |
|---|---|---|---|---|---|---|---|
| Example 7 | A4 | C2 | 120°C x 60 sec. | Not diluted | 80 mJ/cm² | 0.24 µm | ○ |
| Example 8 | A5 | C2 | 120°C x 60 sec. | Not diluted | 70 mJ/cm² | 0.23 µm | ○ |
| Example 9 | A6 | C2 | 120°C x 60 sec. | Not diluted | 60mJ/cm² | 0.22 µm | ○ |
| Example 10 | A7 | C2 | 120°C x 60 sec. | Not diluted | 75 muj/cm² | 0.24 µm | ○ |
| (Footnote of Table 1) Quencher C1: N-phenyldiethanolamine C2: 2,6-diisopropylaniline | | | | | | | |

As shown in Table 1 and 2, the resist composition of the present invention does not cause peeling at the time of developing even if the concentration of the developer is high, and is superior in adhesion to the substrate. The resolution is also improved and the sensitivity is not drastically deteriorated. Each of compositions used in Examples 1 to 6 gives a resist pattern having excellent performances even by the exposure using an ArF excimer laser aligner.

The chemical amplification type positive resist composition of the present invention is superior in adhesion to a substrate and is also superior in various resist performances such as resolution. Accordingly, this composition is suitable for exposure using KrF excimer laser, ArF excimer laser, and the like, thereby to obtain a resist pattern having high performance.

## Claims

1. A chemical amplification type positive resist composition comprising a resin having a butyrolactone residue which may be optionally substituted with an alkyl group and a group capable of cleaving by action of an acid, and an acid generator.

2. The composition according to claim 1, wherein the resin has an alicyclic ring, in addition to the butyrolactone residue and the group capable of cleaving by action of an acid.

3. The composition according to claim 1, wherein the butyrolactone residue is linked to the resin base via an ester or ether linkage.

4. The composition according to claim 1, wherein the butyrolactone residue is an a-residue of butyrolactone.

5. The composition according to claim 1, wherein the group capable of cleaving by action of an acid has an alicyclic ring.

## Patentansprüche

1. Positiv arbeitende Resistzusammensetzung des chemischen Verstärkungstyps, umfassend ein Harz mit einem Butyrolactonrest, der gegebenenfalls mit einem Alkylrest substituiert sein kann, und einem durch Wirkung einer Säure abspaltbaren Rest und einen Säuregenerator.

2. Zusammensetzung nach Anspruch 1, in der das Harz einen alicyclischen Ring zusätzlich zum Butyrolactonrest und dem durch Wirkung einer Säure abspaltbaren Rest aufweist.

3. Zusammensetzung nach Anspruch 1, in der der Butyrolactonrest an den Harzgrundstoff über eine Ester- oder Etherbindung gebunden ist.

4. Zusammensetzung nach Anspruch 1, in der der Butyrolactonrest ein a-Rest von Butyrolacton ist.

5. Zusammensetzung nach Anspruch 1, in der der durch Wirkung einer Säure abspaltbare Rest einen alicyclischen Ring aufweist.

## Revendications

1. Composition pour réserve positive du type amplification chimique comprenant une résine ayant un reste butyrolactone qui peut être éventuellement substitué par un groupe alkyle et un groupe pouvant être clivé par action d'un acide, et un générateur d'acide.

2. Composition selon la revendication 1, caractérisée en ce que la résine possède un cycle alicyclique, en plus du reste butyrolactone et du groupe pouvant être clivé par action d'un acide.

3. Composition selon la revendication 1, caractérisée en ce que le reste butyrolactone est lié à la résine via une liaison ester ou éther.

4. Composition selon la revendication 1, caractérisée en ce que le reste butyrolactone est un reste a de butyrolactone.

5. Composition selon la revendication 1, caractérisée en ce que le groupe pouvant se cliver par action d'un acide a un cycle alicyclique.
